(19) Europäisches Patentamt
European Patent Office
Office européen des brevets

(11) EP 0 819 423 B1

(12) **FASCICULE DE BREVET EUROPEEN**

(45) Date de publication et mention de la délivrance du brevet:
**16.12.1998 Bulletin 1998/51**

(51) Int. Cl.$^6$: **A61K 7/13**

(21) Numéro de dépôt: **97401539.8**

(22) Date de dépôt: **01.07.1997**

(54) **Compositions pour la teinture d'oxydation des fibres kératiniques et procédé de teinture les mettant en oeuvre**

Zusammensetzungen für die Oxydationsfärbung von keratinischen Fasern und Verfahren zur Färbung unter ihrer Verwendung

Composition for the oxydative dyeing of keratinic fibers and dyeing process applying them

(84) Etats contractants désignés:
**AT BE CH DE DK ES FI FR GB GR IE IT LI LU MC NL PT SE**

(30) Priorité: **19.07.1996 FR 9609107**

(43) Date de publication de la demande:
**21.01.1998 Bulletin 1998/04**

(73) Titulaire: **L'OREAL**
**75008 Paris (FR)**

(72) Inventeur:
**Audousset, Marie-Pascale**
**92600 Asnieres (FR)**

(74) Mandataire: **Miszputen, Laurent**
**L'OREAL**
**Département Propriété Industrielle**
**Centre Charles Zviak**
**90, rue du Général Roguet**
**92583 Clichy Cédex (FR)**

(56) Documents cités:

| | |
|---|---|
| **CH-A- 464 448** | **DE-A- 2 714 831** |
| **DE-A- 2 739 227** | **DE-A- 4 408 506** |

- **CHEMICAL ABSTRACTS, vol. 87, no. 10, 5 septembre 1977 Columbus, Ohio, US; abstract no. 73239r, page 325; colonne I; XP002023832 & JP 07 734 082 A (KOEI CHEMICAL LTD.) 15 mars 1977**
- **CHEMICAL ABSTRACTS, vol. 83, no. 18, 3 novembre 1975 Columbus, Ohio, US; abstract no. 152190p, page 322; colonne I; XP002023833 & JP 07 524 401 A (KOEI CHEMICAL CO., LTD.) 15 mars 1975**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.16.6/3.4

## Description

La présente invention a pour objet une composition pour la teinture d'oxydation des fibres kératiniques, en particulier des fibres kératiniques humaines telles que les cheveux, comprenant de la 2-amino 3-hydroxy pyridine à titre de premier coupleur en association avec une base d'oxydation convenablement sélectionnée ainsi qu'un dérivé de méta-aminophénol convenablement sélectionné à titre de second coupleur, ainsi que le procédé de teinture mettant en oeuvre cette composition avec un agent oxydant.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés indoliques comme le 4-hydroxyindole.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans la demande de brevet allemand DE 4 408 506, des compositions pour la teinture d'oxydation des fibres kératiniques, renfermant de la 2-amino 3-hydroxy pyridine à titre de premier coupleur en association avec une base d'oxydation telle que la paratoluylènediamine, et du méta-aminophénol à titre de second coupleur. De telles compositions ne sont cependant pas entièrement satisfaisantes, notamment au niveau de la puissance des colorations obtenues.

Il a également déjà été proposé, notamment dans le brevet américain US 4 421 833, des compositions pour la teinture d'oxydation des fibres kératiniques, renfermant une base d'oxydation particulière, à savoir le 2-(2'-hydroxyéthyl)amino 5-amino benzène, en association avec du 2-méthyl 5-amino phénol et de la 2-amino 3-hydroxy pyridine à titre de coupleurs. De telles compositions ne sont pas non plus entièrement satisfaisantes, notamment au niveau de la tenue des colorations obtenues vis à vis de la transpiration et des agents extérieurs susmentionnés.

Or, la demanderesse vient maintenant de découvrir qu'il est possible d'obtenir de nouvelles teintures particulièrement résistantes, notamment à la transpiration, à la lumière et aux shampooings, en associant de la 2-amino 3-hydroxy pyridine à titre de premier coupleur, au moins une base d'oxydation convenablement sélectionnée et un méta-aminophénol convenablement sélectionné à titre de second coupleur.

Cette découverte est à la base de la présente invention.

L'invention a donc pour objet une composition pour la teinture d'oxydation des fibres kératiniques et en particulier des fibres kératiniques humaines telles que les cheveux, caractérisée par le fait qu'elle comprend, dans un milieu approprié pour la teinture :

- à titre de premier coupleur, de la 2-amino 3-hydroxy pyridine et/ou au moins l'un de ses sels d'addition avec un acide,
- au moins une base d'oxydation choisie parmi

(i) les paraphénylènediamines de formule (I) suivante, et leurs sels d'addition avec un acide :

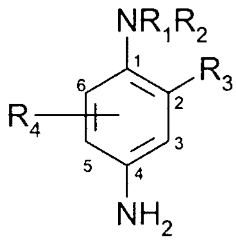

(I)

dans laquelle :

$R_1$ représente un atome d'hydrogène, un radical alkyle en $C_1$-$C_4$, monohydroxyalkyle en $C_1$-$C_4$, polyhydroxyalkyle en $C_2$-$C_4$, alcoxyalkyle ayant ≤ 4 atomes de carbone, phényle ou 4'-aminophényle,
$R_2$ représente un atome d'hydrogène, un radical alkyle en $C_1$-$C_4$, monohydroxyalkyle en $C_1$-$C_4$ ou polyhydroxyalkyle en $C_2$-$C_4$,
$R_3$ représente un atome d'hydrogène, un atome d'halogène tel qu'un atome de chlore, un radical alkyle en $C_1$-$C_4$, monohydroxyalkyle en $C_1$-$C_4$ ou hydroxyalcoxy en $C_1$-$C_4$,
$R_4$ représente un atome d'hydrogène ou un radical alkyle en $C_1$-$C_4$,

sous réserve que :

- lorsque $R_2$ représente un radical β-hydroxyéthyle, et que $R_1$ et $R_4$ représentent un atome d'hydrogène, alors $R_3$ est différent d'un radical méthyle,
- lorsque $R_2$ représente un radical β-hydroxyéthyle, et que $R_1$ et $R_3$ représentent un atome d'hydrogène, alors $R_4$ ne peut représenter un radical méthyle en position 6,

(ii) les para-aminophénols de formule (II) suivante, et leurs sels d'addition avec un acide :

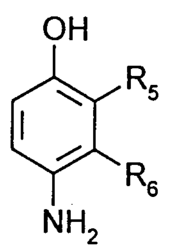

(II)

dans laquelle :

$R_5$ représente un atome d'hydrogène, un radical alkyle en $C_1$-$C_4$, monohydroxyalkyle en $C_1$-$C_4$, alcoxyalkyle en $C_1$-$C_4$ ou aminoalkyle en $C_1$-$C_4$,
$R_6$ représente un atome d'hydrogène ou de fluor, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxyalkyle en $C_1$-$C_4$,
sous réserve qu'au moins un des radicaux $R_5$ ou $R_6$ représente un atome d'hydrogène,

- à titre de second coupleur, au moins un méta-aminophénol de formule (III) suivante et/ou au moins l'un de leurs sels d'addition avec acide :

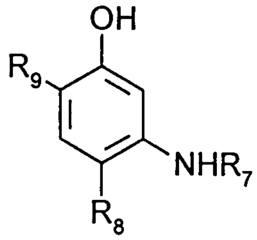

(III)

dans laquelle :

- $R_7$ représente un atome d'hydrogène, un radical alkyle en $C_1$-$C_4$, monohydroxyalkyle en $C_1$-$C_4$ ou polyhydroxyalkyle en $C_2$-$C_4$,
- $R_8$ représente un atome d'hydrogène, un radical alkyle en $C_1$-$C_4$, alcoxy en $C_1$-$C_4$ ou un atome d'halogène choisi parmi le chlore, le brome ou le fluor,
- $R_9$ représente un atome d'hydrogène, un radical alkyle en $C_1$-$C_4$, alcoxy en $C_1$-$C_4$, monohydroxyalkyle en $C_1$-$C_4$, polyhydroxyalkyle en $C_2$-$C_4$, monohydroxyalcoxy en $C_1$-$C_4$ ou polyhydroxyalcoxy en $C_2$-$C_4$,

étant entendu qu'au moins un des radicaux $R_7$, $R_8$ et $R_9$ est différent d'un atome d'hydrogène.

Les colorations obtenues avec la composition conforme à l'invention présentent une bonne puissance tinctoriale et d'excellentes propriétés de résistance à la fois vis à vis des agents atmosphériques tels que la lumière et les intempéries et vis à vis de la transpiration et des différents traitements que peuvent subir les cheveux (lavages, déformations permanentes). Ces propriétés sont particulièrement remarquables notamment en ce qui concerne la résistance des colorations obtenues vis à vis de la transpiration, de la lumière et des shampooings.

L'invention a également pour objet un procédé de teinture d'oxydation des fibres kératiniques mettant en oeuvre cette composition.

Les sels d'addition avec un acide utilisables dans le cadre des compositions tinctoriales de l'invention (bases et coupleurs) sont notamment choisis parmi les chlorhydrates, les bromhydrates, les sulfates et les tartrates.

Parmi les paraphénylènediamines de formule (I) ci-dessus, on peut plus particulièrement citer la paraphénylènediamine, la paratoluylènediamine, la 2-n-propyl paraphénylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, le 1-β-méthoxyéthylamino 4-aminobenzène, et leurs sels d'addition avec un acide.

Parmi les para-aminophénols de formule (II) ci-dessus, on peut plus particulièrement citer le para-aminophénol, le 3-méthyl 4-aminophénol, le 3-fluoro 4-aminophénol, le 3-hydroxyméthyl 4-aminophénol, le 2-méthyl 4-aminophénol, le 2-hydroxyméthyl 4-aminophénol, le 2-méthoxyméthyl 4-aminophénol, le 2-aminométhyl 4-aminophénol, le 2-(β-hydroxyéthyl aminométhyl) 4-aminophénol, et leurs sels d'addition avec un acide.

Parmi les méta-aminophénols de formule (III) ci-dessus, on peut plus particulièrement citer le 5-amino 2-méthoxy phénol, le 5-amino 2-(β-hydroxyéthyloxy) phénol, le 5-amino 2-méthyl phénol, le 5-N-(β-hydroxyéthyl)amino 2-méthyl phénol, le 5-N-(β-hydroxyéthyl)amino 4-méthoxy 2-méthyl phénol, le 5-amino 4-méthoxy 2-méthyl phénol, le 5-amino 4-chloro 2-méthyl phénol, le 5-amino 2,4-diméthoxy phénol, le 5-(γ-hydroxypropylamino) 2-méthyl phénol, et leurs sels d'addition avec un acide.

La 2-amino 3-hydroxy pyridine et/ou le ou les sels d'addition de ce composé avec un acide représentent de préférence de 0,0001 à 10 % en poids environ du poids total de la composition tinctoriale et encore plus préférentiellement de 0,005 à 7 % en poids environ.

L'ensemble des bases d'oxydation conformes à l'invention, c'est à dire le ou les paraphénylènediamines de formule (I) et/ou le ou les para-aminophénols de formule (II), représente de préférence de 0,0005 à 10 % en poids environ du poids total de la composition tinctoriale et encore plus préférentiellement de 0,01 à 7 % en poids environ.

Le ou les méta-aminophénols de formule (III) conformes à l'invention représentent de préférence de 0,0001 à 5 % en poids environ du poids total de la composition tinctoriale et encore plus préférentiellement de 0,001 à 3 % en poids environ.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; le glycérol ; les glycols et éthers de glycols comme le 2-butoxyéthanol, le propylèneglycol, le monoéthyléther et le

monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale telle que définie précédemment est généralement compris entre 5 et 12 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemples, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants, on peut citer, à titre d'exemples, l'ammoniaque, les carbonates alcalins, les alcanolamines telles que les mono-, di- et triéthanolamines ainsi que leurs dérivés, les hydroxydes de sodium ou de potassium et les composés de formule (IV) suivante :

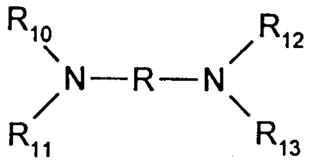

(IV)

dans laquelle R est un reste propylène éventuellement substitué par un groupement hydroxyle ou un radical alkyle en $C_1$-$C_4$ ; $R_{10}$, $R_{11}$, $R_{12}$ et $R_{13}$, identiques ou différents, représentent un atome d'hydrogène, un radical alkyle en $C_1$-$C_4$ ou hydroxyalkyle en $C_1$-$C_4$.

La composition tinctoriale conforme à l'invention peut encore contenir, en plus des colorants définis ci-dessus, d'autres coupleurs différents de la 2-amino 3-hydroxy pyridine et des méta-aminophénols de formule (III) et de leurs sels d'addition avec un acide et/ou d'autres bases d'oxydation différentes des paraphénylènediamines de formule (I), des para-aminophénols de formule (II) et de leurs sels d'addition avec un acide et/ou des colorants directs, notamment pour modifier les nuances ou les enrichir en reflets.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres à colorer la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 40 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 2 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

**EXEMPLES**

**<u>EXEMPLES 1 et 2</u>**

On a préparé les compositions tinctoriales conformes à l'invention, suivantes (teneurs en grammes) :

| EXEMPLE | 1 | 2 |
|---|---|---|
| Sulfate de paratoluylènediamine (Base d'oxydation) | 0,434 | - |
| Paratoluylènediamine (Base d'oxydation) | - | 1,0 |
| 2-amino 3-hydroxy pyridine (premier coupleur) | 0,11 | 0,5 |
| 2-méthyl 5-aminophénol (second coupleur) | 0,123 | 0,56 |
| Support de teinture commun (*) | (*) | (*) |
| Eau q.s.p. | 100 g | 100 g |

(*) support de teinture commun :

| | | |
|---|---|---|
| - Alcool oléique polyglycérolé à 2 moles de glycérol | 4,0 | g |
| - Alcool oléique polyglycérolé à 4 moles de glycérol, à 78 % de matières actives (M.A.) | 5,69 | g M.A. |
| - Acide oléique | 3,0 | g |
| - Amine oléique à 2 moles d'oxyde d'éthylène vendue sous la dénomination commerciale ETHOMEEN O12 par la société AKZO | 7,0 | g |
| - Laurylamino succinamate de diéthylaminopropyle, sel de sodium à 55 % de M.A. | 3,0 | g M.A. |
| - Alcool oléique | 5,0 | g |
| - Diéthanolamide d'acide oléique | 12,0 | g |
| - Propylèneglycol | 3,5 | g |

| | |
|---|---|
| - Alcool éthylique | 7,0 g |
| - Dipropylèneglycol | 0,5 g |
| - Monométhyléther de propylèneglycol | 9,0 g |
| - Métabisulfite de sodium en solution aqueuse, à 35 % de M.A. | 0,455 g M.A. |
| - Acétate d'ammonium | 0,8 g |
| - Antioxydant, séquestrant | q.s. |
| - Parfum, conservateur | q.s. |
| - Ammoniaque à 20 % de $NH_3$ | 10,0 g |

Au moment de l'emploi, on a mélangé chaque composition tinctoriale avec une quantité égale d'une composition oxydante constituée par une solution d'eau oxygénée à 20 volumes (6 % en poids) et présentant un pH d'environ 3.

Chaque mélange obtenu présentait un pH d'environ 10,2 et a été appliqué pendant 30 minutes sur des mèches de cheveux gris à 90 % de blancs, naturels ou permanentés. Les mèches de cheveux ont ensuite été rincées, lavées avec un shampooing standard puis séchées.

Les mèches de cheveux ont été teintes dans les nuances figurant dans le tableau ci-dessous :

| EXEMPLE | NUANCE SUR CHEVEUX NATURELS | NUANCE SUR CHEVEUX PERMANENTES |
|---|---|---|
| 1 | Acajou irisé | Rose rabattu |
| 2 | Acajou | Violine légèrement rabattu |

**<u>EXEMPLES 3 et 4 COMPARATIFS</u>**

On a préparé les compositions tinctoriales suivantes (teneurs en grammes):

| EXEMPLE | 3 (**) | 4 |
|---|---|---|
| Sulfate de paratoluylènediamine (Base d'oxydation) | - | 0,434 |
| Sulfate, dihydrate de 4-amino 1-N-(β-hydroxyéthyl)amino 2-méthyl benzène (Base d'oxydation) | 0,609 | - |
| 2-amino 3-hydroxy pyridine (premier coupleur) | 0,11 | 0,11 |
| 2-méthyl 5-aminophénol (second coupleur) | 0,123 | 0,123 |
| Support de teinture commun (*) | (*) | (*) |
| Eau q.s.p. | 100 g | 100 g |

(*) <u>support de teinture commun</u> :
Il est identique à celui utilisé dans les exemples 1 et 2 ci-dessus.
(**) : exemple ne faisant pas partie de l'invention

Il est important de noter que les compositions tinctoriales ci-dessus contiennent la même quantité molaire de base d'oxydation, à savoir $2.10^{-3}$ mole.

Au moment de l'emploi, on a mélangé chaque composition tinctoriale avec une quantité égale d'une composition oxydante constituée par une solution d'eau oxygénée à 20 volumes (6 % en poids) et présentant un pH d'environ 3.

Chaque mélange obtenu présentait un pH d'environ 10,2 et a été appliqué sur des mèches de cheveux gris naturels à 90 % de blancs selon le procédé de teinture décrit ci-dessus dans les exemples 1 et 2.

Les mèches ainsi teintes ont ensuite subi un test de résistance à la lumière (Xenotest).

Pour ce faire, les mèches de cheveux teintes ont été fixées sur un support (carton ou plastique). Ces supports ont été disposés sur des porte-échantillons que l'on a fait tourner autour d'une lampe Xénon pendant une durée de 40 heures sous un taux d'humidité relative de 25 ± 5 % et à une température de 42,5 ± 2,5°C.

La couleur des mèches a été évaluée dans le système MUNSELL, avant et après le test de résistance à la lumière, au moyen d'un colorimètre CM 2002 MINOLTA.

Selon la notation MUNSELL, une couleur est définie par l'expression **H V / C** dans laquelle les trois paramètres désignent respectivement la teinte ou Hue (**H**), l'intensité ou Value (**V**) et la pureté ou Chromaticité (**C**), la barre oblique de cette expression est simplement une convention et n'indique pas un ratio.

La différence de couleur de chaque mèche avant et après le test de résistance à la lumière reflète la dégradation de la coloration due à l'action de la lumière et a été calculée en appliquant la formule de NICKERSON :

$$\Delta E = 0{,}4\ Co\Delta H + 6\Delta V + 3\ \Delta C,$$

telle que décrite par exemple dans "Couleur, Industrie et Technique"; pages 14-17 ; vol. n° 5; 1978.

Dans cette formule, **ΔE** représente la différence de couleur entre deux mèches, **ΔH**, **ΔV** et **ΔC** représentent la variation en valeur absolue des paramètres **H**, **V** et **C**, et **Co** représente la pureté de la mèche par rapport à laquelle on désire évaluer la différence de couleur (pureté de la mèche avant le test).

Les résultats sont donnés dans le tableau ci-dessous :

| EXEMPLE | Couleur avant le test | Couleur après le test | Dégradation de la coloration | | | |
|---|---|---|---|---|---|---|
| | | | ΔH | ΔV | ΔC | ΔE |
| 3 (**) | 8,6 RP 3,7 / 1,8 | 4,9 R 4,3 / 1,7 | 6,3 | 0,6 | 0,1 | **8,4** |
| 4 | 4,5 R 3,5 / 2,6 | 7,0 R 3,5 / 2,5 | 2,5 | 0 | 0,1 | **2,9** |

(**) : exemple ne faisant pas partie de l'invention

On constate que la coloration obtenue avec la composition tinctoriale de l'exemple 4 renfermant une association conforme à l'invention de la 2-amino 3-hydroxy pyridine, du 2-méthyl 5-amino phénol et de la paratoluylènediamine (paraphénylènediamine de formule (I)) résiste beaucoup mieux à l'action de la lumière que la coloration obtenue avec la composition tinctoriale de l'exemple 3 ne faisant pas partie de l'invention car contenant l'association de la 2-amino 3-hydroxy pyridine, du 2-méthyl 5-amino phénol et du 4-amino 1-N-(β-hydroxyéthyl)amino 2-méthyl benzène qui est un composé ne répondant pas à la formule (I) des paraphénylènediamines définie précédemment, et telle que décrite par exemple dans le brevet US 5 421 833.

## Revendications

1. Composition pour la teinture d'oxydation des fibres kératiniques et en particulier des fibres kératiniques humaines telles que les cheveux, caractérisée par le fait qu'elle comprend, dans un milieu approprié pour la teinture :

   - à titre de premier coupleur, de la 2-amino 3-hydroxy pyridine et/ou au moins l'un de ses sels d'addition avec un acide,

   - au moins une base d'oxydation choisie parmi

     (i) les paraphénylènediamines de formule (I) suivante, et leurs sels d'addition avec un acide :

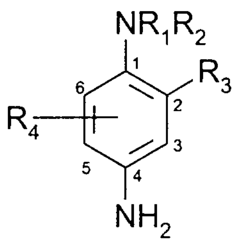

(I)

dans laquelle :

$R_1$ représente un atome d'hydrogène, un radical alkyle en $C_1$-$C_4$, monohydroxyalkyle en $C_1$-$C_4$, polyhydroxyalkyle en $C_2$-$C_4$, alcoxyalkyle ayant ≤ 4 atomes de carbone, phényle ou 4'-aminophényle,
$R_2$ représente un atome d'hydrogène, un radical alkyle en $C_1$-$C_4$, monohydroxyalkyle en $C_1$-$C_4$ ou polyhydroxyalkyle en $C_2$-$C_4$,
$R_3$ représente un atome d'hydrogène, un atome d'halogène tel qu'un atome de chlore, un radical alkyle en $C_1$-$C_4$, monohydroxyalkyle en $C_1$-$C_4$ ou hydroxyalcoxy en $C_1$-$C_4$,
$R_4$ représente un atome d'hydrogène ou un radical alkyle en $C_1$-$C_4$,

sous réserve que :

- lorsque $R_2$ représente un radical β-hydroxyéthyle, et que $R_1$ et $R_4$ représentent un atome d'hydrogène, alors $R_3$ est différent d'un radical méthyle,
- lorsque $R_2$ représente un radical β-hydroxyéthyle, et que $R_1$ et $R_3$ représentent un atome d'hydrogène, alors $R_4$ ne peut représenter un radical méthyle en position 6,

(ii) les para-aminophénols de formule (II) suivante, et leurs sels d'addition avec un acide :

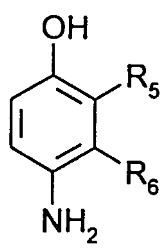

(II)

dans laquelle :

$R_5$ représente un atome d'hydrogène, un radical alkyle en $C_1$-$C_4$, monohydroxyalkyle en $C_1$-$C_4$, alcoxyalkyle ayant ≤ 4 atomes de carbone ou aminoalkyle en $C_1$-$C_4$,
$R_6$ représente un atome d'hydrogène ou de fluor, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxyalkyle en $C_1$-$C_4$, sous réserve qu'au moins un des radicaux $R_5$ ou $R_6$ représente un atome d'hydrogène,

- à titre de second coupleur, au moins un méta-aminophénol de formule (III) suivante et/ou au moins l'un de leurs sels d'addition avec acide :

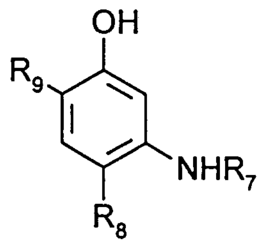

(III)

dans laquelle :

- $R_7$ représente un atome d'hydrogène, un radical alkyle en $C_1$-$C_4$, monohydroxyalkyle en $C_1$-$C_4$ ou polyhydroxyalkyle en $C_2$-$C_4$,
- $R_8$ représente un atome d'hydrogène, un radical alkyle en $C_1$-$C_4$, alcoxy en $C_1$-$C_4$ ou un atome d'halogène choisi parmi le chlore, le brome ou le fluor,
- $R_9$ représente un atome d'hydrogène, un radical alkyle en $C_1$-$C_4$, alcoxy en $C_1$-$C_4$, monohydroxyalkyle en $C_1$-$C_4$, polyhydroxyalkyle en $C_2$-$C_4$, monohydroxyalcoxy en $C_1$-$C_4$ ou polyhydroxyalcoxy en $C_2$-$C_4$,

étant entendu qu'au moins un des radicaux $R_7$, $R_8$ et $R_9$ est différent d'un atome d'hydrogène.

**2.** Composition selon la revendication 1, caractérisée par le fait que les paraphénylènediamines de formule (I) sont choisies parmi la paraphénylènediamine, la paratoluylènediamine, la 2-n-propyl paraphénylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, le 1-β-méthoxyéthylamino 4-aminobenzène, et leurs sels d'addition avec un acide.

**3.** Composition selon l'une quelconque des revendications précédentes, caractérisée par le fait que les para-aminophénols de formule (II) sont choisis parmi le para-aminophénol, le 3-méthyl 4-aminophénol, le 3-fluoro 4-aminophénol, le 3-hydroxyméthyl 4-aminophénol, le 2-méthyl 4-aminophénol, le 2-hydroxyméthyl 4-aminophénol, le 2-méthoxyméthyl 4-aminophénol, le 2-aminométhyl 4-aminophénol, le 2-(β-hydroxyéthyl aminométhyl) 4-aminophénol, et leurs sels d'addition avec un acide.

**4.** Composition selon l'une quelconque des revendications précédentes, caractérisée par le fait que les méta-aminophénols de formule (III) sont choisis parmi le 5-amino 2-méthoxy phénol, le 5-amino 2-(β-hydroxyéthyloxy) phénol, le 5-amino 2-méthyl phénol, le 5-N-(β-hydroxyéthyl)amino 2-méthyl phénol, le 5-N-(β-hydroxyéthyl)amino 4-méthoxy 2-méthyl phénol, le 5-amino 4-méthoxy 2-méthyl phénol, le 5-amino 4-chloro 2-méthyl phénol, le 5-amino 2,4-diméthoxy phénol, le 5-(γ-hydroxypropylamino) 2-méthyl phénol, et leurs sels d'addition avec un acide.

**5.** Composition selon l'une quelconque des revendications précédentes, caractérisée par le fait que les sels d'addition avec un acide sont choisis parmi les chlorhydrates, les bromhydrates, les sulfates et les tartrates.

**6.** Composition selon l'une quelconque des revendications précédentes, caractérisée par le fait que la 2-amino 3-hydroxy pyridine et/ou le ou les sels d'addition de ce composé avec un acide représentent de 0,0001 à 10 % en poids du poids total de la composition tinctoriale.

**7.** Composition selon la revendication 6, caractérisée par le fait que la 2-amino 3-hydroxy pyridine et/ou le ou les sels d'addition de ce composé avec un acide représentent de 0,005 à 7 % en poids du poids total de la composition tinctoriale.

**8.** Composition selon l'une quelconque des revendications précédentes, caractérisée par le fait que la et/ou les paraphénylènediamines de formule (I) et/ou le et/ou les para-aminophénols de formule (II) représentent de 0,0005 à 10 % en poids du poids total de la composition tinctoriale.

**9.** Composition selon la revendication 8, caractérisée par le fait que la et/ou les paraphénylènediamines de formule

(I) et/ou le et/ou les para-aminophénols de formule (II) représentent de 0,01 à 7 % en poids du poids total de la composition tinctoriale.

**10.** Composition selon l'une quelconque des revendications précédentes, caractérisée par le fait que le ou les méta-aminophénols de formule (III) représentent de 0,0001 à 5 % en poids du poids total de la composition tinctoriale.

**11.** Composition selon la revendication 10, caractérisée par le fait que le ou les méta-aminophénols formule (III) représentent de 0,001 à 3 % en poids du poids total de la composition tinctoriale.

**12.** Composition selon l'une quelconque des revendications précédentes, caractérisée par le fait que ledit milieu approprié pour la teinture est constitué par de l'eau ou par un mélange d'eau et d'au moins un solvant organique choisi parmi les alcanols inférieurs en $C_1$-$C_4$, le glycérol, les glycols et éthers de glycols, les alcools aromatiques, les produits analogues et leurs mélanges.

**13.** Composition selon l'une quelconque des revendications précédentes, caractérisée par le fait qu'elle présente un pH compris entre 5 et 12.

**14.** Composition selon l'une quelconque des revendications précédentes, caractérisée par le fait qu'elle contient, en outre, d'autres coupleurs différents de la 2-amino 3-hydroxy pyridine, des méta-aminophénols de formule (III) et de leurs sels d'addition avec un acide et/ou d'autres bases d'oxydation différentes des paraphénylènediamines de formule (I), des para-aminophénols de formule (II) et de leurs sels d'addition avec un acide et/ou des colorants directs.

**15.** Procédé de teinture des fibres kératiniques et en particulier des fibres kératiniques humaines telles que les cheveux caractérisé par le fait que l'on applique sur ces fibres une composition tinctoriale telle que définie à l'une quelconque des revendications 1 à 14 et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

**16.** Procédé selon la revendication 15, caractérisé par le fait que l'agent oxydant présent dans la composition oxydante est choisi parmi le peroxyde d'hydrogène, le peroxyde d'urée, les bromates de métaux alcalins, les persels tels que les perborates et persulfates.

**17.** Dispositif à plusieurs compartiments ou "kit" de teinture à plusieurs compartiments dont un premier compartiment renferme une composition tinctoriale telle que définie à l'une quelconque des revendications 1 à 14 et un second compartiment renferme une composition oxydante comprenant un agent oxydant.

**Claims**

**1.** Composition for the oxidation dyeing of keratin fibres, and in particular of human keratin fibres such as the hair, characterized in that it comprises, in a medium which is suitable for dyeing:

- as first coupler, 2-amino-3-hydroxypyridine and/or at least one of the addition salts thereof with an acid,
- at least one oxidation base chosen from

(i) para-phenylenediamines of formula (I) below, and the addition salts thereof with an acid:

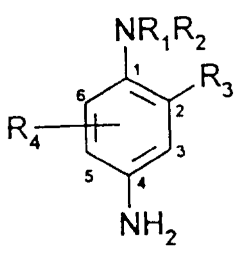

(I)

in which:

$R_1$ represents a hydrogen atom or a $C_1$-$C_4$ alkyl, $C_1$-$C_4$ monohydroxyalkyl, $C_2$-$C_4$ polyhydroxyalkyl, alkoxyalkyl having ≤ 4 carbon atoms, phenyl or 4'-aminophenyl radical,

$R_2$ represents a hydrogen atom or a $C_1$-$C_4$ alkyl, $C_1$-$C_4$ monohydroxyalkyl or $C_2$-$C_4$ polyhydroxyalkyl radical,

$R_3$ represents a hydrogen atom, a halogen atom such as a chlorine atom, or a $C_1$-$C_4$ alkyl, $C_1$-$C_4$ monohydroxyalkyl or $C_1$-$C_4$ hydroxyalkoxy radical,

$R_4$ represents a hydrogen atom or a $C_1$-$C_4$ alkyl radical,

with the proviso that:

- when $R_2$ represents a β-hydroxyethyl radical and when $R_1$ and $R_4$ represent a hydrogen atom, then $R_3$ is other than a methyl radical,
- when $R_2$ represents a β-hydroxyethyl radical and when $R_1$ and $R_3$ represent a hydrogen atom, then $R_4$ cannot represent a methyl radical in position 6,

(ii) para-aminophenols of formula (II) below, and the addition salts thereof with an acid:

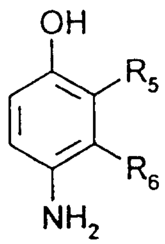

(II)

in which:

$R_5$ represents a hydrogen atom or a $C_1$-$C_4$ alkyl, $C_1$-$C_4$ monohydroxyalkyl, alkoxyalkyl having ≤ 4 carbon atoms or $C_1$-$C_4$ aminoalkyl radical,

$R_6$ represents a hydrogen or fluorine atom or a $C_1$-$C_4$ alkyl, $C_1$-$C_4$ monohydroxyalkyl, $C_2$-$C_4$ polyhydroxyalkyl, $C_1$-$C_4$ aminoalkyl, cyano($C_1$-$C_4$)alkyl or alkoxy($C_1$-$C_4$)alkyl radical, with the proviso that at least one of the radicals $R_5$ or $R_6$ represents a hydrogen atom,

- as second coupler, at least one meta-aminophenol of formula (III) below and/or at least one of the addition salts thereof with an acid:

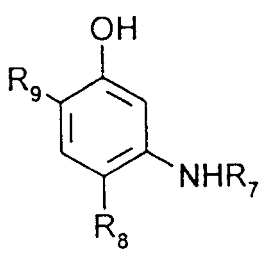

(III)

in which:

- $R_7$ represents a hydrogen atom or a $C_1$-$C_4$ alkyl, $C_1$-$C_4$ monohydroxyalkyl or $C_2$-$C_4$ polyhydroxyalkyl radical,
- $R_8$ represents a hydrogen atom, a $C_1$-$C_4$ alkyl or $C_1$-$C_4$ alkoxy radical or a halogen atom chosen from chlorine, bromine and fluorine,
- $R_9$ represents a hydrogen atom or a $C_1$-$C_4$ alkyl, $C_1$-$C_4$ alkoxy, $C_1$-$C_4$ monohydroxyalkyl, $C_2$-$C_4$ polyhydroxyalkyl, $C_1$-$C_4$ monohydroxyalkoxy or $C_2$-$C_4$ polyhydroxyalkoxy radical,

it being understood that at least one of the radicals $R_7$, $R_8$ and $R_9$ is other than a hydrogen atom.

2. Composition according to Claim 1, characterized in that the para-phenylenediamines of formula (I) are chosen from para-phenylenediamine, para-toluylenediamine, 2-n-propyl-para-phenylenediamine, 2-isopropyl-para-phenylenediamine, 2-β-hydroxyethyl-para-phenylenediamine, 2-β-hydroxyethyloxy-para-phenylenediamine, 2,6-dimethyl-para-phenylenediamine, 2,6-diethyl-para-phenylenediamine, 2,3-dimethyl-para-phenylenediamine, N,N-bis(β-hydroxyethyl)-para-phenylenediamine and 1,β-methoxyethylamino-4-aminobenzene, and the addition salts thereof with an acid.

3. Composition according to either of the preceding claims, characterized in that the para-aminophenols of formula (II) are chosen from para-aminophenol, 3-methyl-4-aminophenol, 3-fluoro-4-aminophenol, 3-hydroxymethyl-4-aminophenol, 2-methyl-4-aminophenol, 2-hydroxymethyl-4-aminophenol, 2-methoxymethyl-4-aminophenol, 2-aminomethyl-4-aminophenol and 2-(β-hydroxyethylaminomethyl)-4-aminophenol, and the addition salts thereof with an acid.

4. Composition according to any one of the preceding claims, characterized in that the meta-aminophenols of formula (III) are chosen from 5-amino-2-methoxyphenol, 5-amino-2-(β-hydroxyethyloxy)phenol, 5-amino-2-methylphenol, 5-N-(β-hydroxyethyl)amino-2-methylphenol, 5-N-(β-hydroxyethyl)amino-4-methoxy-2-methylphenol, 5-amino-4-methoxy-2-methylphenol, 5-amino-4-chloro-2-methylphenol, 5-amino-2,4-dimethoxyphenol and 5-(γ-hydroxypropylamino)-2-methylphenol, and the addition salts thereof with an acid.

5. Composition according to any one of the preceding claims, characterized in that the addition salts with an acid are chosen from the hydrochlorides, hydrobromides, sulphates and tartrates.

6. Composition according to any one of the preceding claims, characterized in that the 2-amino-3-hydroxypyridine and/or the addition salt or salts of this compound with an acid represent from 0.0001 to 10% by weight relative to the total weight of the dye composition.

7. Composition according to Claim 6, characterized in that the 2-amino-3-hydroxypyridine and/or the addition salt or salts of this compound with an acid represent from 0.005 to 7% by weight relative to the total weight of the dye composition.

8. Composition according to any one of the preceding claims, characterized in that the para-phenylenediamine(s) of formula (I) and/or the para-aminophenol(s) of formula (II) represent from 0.0005 to 10% by weight relative to the total weight of the dye composition.

9. Composition according to Claim 8, characterized in that the para-phenylenediamine(s) of formula (I) and/or the para-aminophenol(s) of formula (II) represent 0.01 to 7% by weight relative to the total weight of the dye composition.

10. Composition according to any one of the preceding claims, characterized in that the meta-aminophenol(s) of formula (III) represent from 0.0001 to 5% by weight relative to the total weight of the dye composition.

11. Composition according to Claim 10, characterized in that the meta-aminophenol(s) of formula (III) represent from 0.001 to 3% by weight relative to the total weight of the dye composition.

12. Composition according to any one of the preceding claims, characterized in that the said medium which is suitable for dyeing consists of water or of a mixture of water and at least one organic solvent chosen from $C_1$-$C_4$ lower alkanols, glycerol, glycols and glycol ethers, aromatic alcohols, similar products and mixtures thereof.

13. Composition according to any one of the preceding claims, characterized in that it has a pH of between 5 and 12.

14. Composition according to any one of the preceding claims, characterized in that it also contains couplers other than 2-amino-3-hydroxypyridine, meta-aminophenols of formula (III) and the addition salts thereof with an acid and/or oxidation bases other than the para-phenylenediamines of formula (I), para-aminophenols of formula (II) and the addition salts thereof with an acid and/or direct dyes.

15. Process for dyeing keratin fibres, and in particular human keratin fibres such as the hair, characterized in that a dye

composition as defined in any one of Claims 1 to 14 is applied to these fibres and the colour is developed at acidic, neutral or alkaline pH using an oxidizing agent which is added, only at the time of use, to the dye composition or which is present in an oxidizing composition that is applied simultaneously or sequentially in a separate manner.

**16.** Process according to Claim 15, characterized in that the oxidizing agent present in the oxidizing composition is chosen from hydrogen peroxide, urea peroxide, alkali metal bromates and persalts such as perborates and persulphates.

**17.** Multi-compartment device or multi-compartment dyeing "kit", a first compartment of which contains a dye composition as defined in any one of Claims 1 to 14, and a second compartment of which contains an oxidizing composition comprising an oxidizing agent.

**Patentansprüche**

**1.** Zusammensetzung zum oxidativen Färben von Keratinfasern und insbesondere menschlichen Keratinfasern, wie dem Haar,
dadurch gekennzeichnet, daß
sie in einem zum Färben geeigneten Medium enthält:

- als ersten Kuppler 2-Amino-3-hydroxy-pyridin und/oder mindestens ein Additionssalz dieser Verbindung mit einer Säure,

- mindestens eine Oxidationsbase, die ausgewählt ist unter:

(i) den p-Phenylendiaminen der folgenden Formel (I) und den Additionssalzen dieser Verbindungen mit einer Säure:

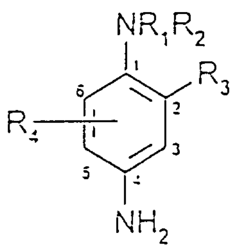

(I),

worin bedeuten:

$R_1$ Wasserstoff, $C_{1-4}$-Alkyl, $C_{1-4}$-Monohydroxyalkyl, $C_{2-4}$-Polyhydroxyalkyl, Alkoxyalkyl mit $\leq$ 4 Kohlenstoffatomen, Phenyl oder 4'-Aminophenyl;

$R_2$ Wasserstoff, $C_{1-4}$-Alkyl, $C_{1-4}$-Monohydroxyalkyl oder $C_{2-4}$-Polyhydroxyalkyl;

$R_3$ Wasserstoff, Halogen, wie Chlor, $C_{1-4}$-Alkyl, $C_{1-4}$-Monohydroxyalkyl oder $C_{1-4}$-Hydroxyalkoxy;

$R_4$ Wasserstoff oder $C_{1-4}$-Alkyl,

mit der Maßgabe, daß

- $R_3$ von Methyl verschieden ist, wenn $R_2$ β-Hydroxyethyl ist und $R_1$ und $R_4$ Wasserstoff bedeuten; und
- $R_4$ nicht Methyl in 6-Stellung bedeuten kann, wenn $R_2$ β-Hydroxyethyl ist und $R_1$ und $R_3$ Wasserstoff bedeuten;

(ii) den p-Aminophenolen der folgenden Formel (II) und den Additionssalzen dieser Verbindungen mit einer Säure:

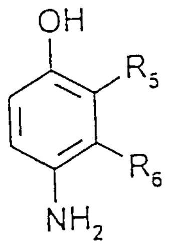

(II)

worin bedeuten:

$R_5$ Wasserstoff, $C_{1-4}$-Alkyl, $C_{1-4}$-Monohydroxyalkyl, Alkoxyalkyl mit ≤ 4 Kohlenstoffatomen oder $C_{1-4}$-Aminoalkyl;

$R_6$ Wasserstoff, Fluor, $C_{1-4}$-Alkyl, $C_{1-4}$-Monohydroxyalkyl, $C_{2-4}$-Polyhydroxyalkyl, $C_{1-4}$-Aminoalkyl, $C_{1-4}$-Cyanoalkyl oder $C_{1-4}$-Alkoxyalkyl;

mit der Maßgabe, daß mindestens eine der Gruppen $R_5$ oder $R_6$ Wasserstoff bedeutet;

- als zweiten Kuppler mindestens ein m-Aminophenol der folgenden Formel (III) und/oder mindestens ein Additionssalz dieser Verbindungen mit einer Säure:

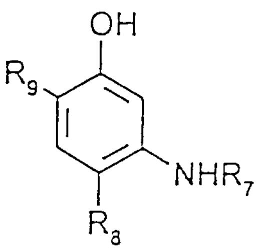

(III),

worin bedeuten:

$R_7$ Wasserstoff, $C_{1-4}$-Alkyl, $C_{1-4}$-Monohydroxyalkyl oder $C_{2-4}$-Polyhydroxyalkyl;

$R_8$ Wasserstoff, $C_{1-4}$-Alkyl, $C_{1-4}$-Alkoxy oder ein Halogenatom, das unter Chlor, Brom oder Fluor ausgewählt ist;

$R_3$ Wasserstoff, $C_{1-4}$-Alkyl, $C_{1-4}$-Alkoxy, $C_{1-4}$-Monohydroxyalkyl, $C_{2-4}$-Polyhydroxyalkyl, $C_{1-4}$-Monohydroxyalkoxy oder $C_{2-4}$-Polyhydroxyalkoxy,

mit der Maßgabe, daß mindestens eine der Gruppen $R_7$, $R_8$ und $R_9$ von Wasserstoff verschieden ist.

**2.** Zusammensetzung nach Anspruch 1, dadurch gekennzeichnet, daß die p-Phenylendiamine der Formel (I) ausgewählt sind unter: p-Phenylendiamin, p-Toluylendiamin, 2-n-Propyl-p-phenylendiamin, 2-Isopropyl-p-phenylendiamin, 2-β-Hydroxyethyl-p-phenylendiamin, 2-β-Hydroxyethyloxy-p-phenylendiamin, 2,6-Dimethyl-p-phenylendiamin, 2,6-Diethyl-p-phenylendiamin, 2,3-Dimethyl-p-phenylendiamin, N,N-Bis-(β-hydroxyethyl)-p-phenylendiamin, 1-β-Methoxyethylamino-4-aminobenzol und den Additionssalzen dieser Verbindungen mit einer Säure.

**3.** Zusammensetzung nach einem der vorhergehenden Ansprüche, dadurch gekennzeichnet, daß die p-Aminophenole der Formel (II) ausgewählt sind unter: p-Aminophenol, 3-Methyl-4-amino-phenol, 3-Fluor-4-amino-phenol, 3-Hydroxymethyl-4-amino-phenol, 2-Methyl-4-amino-phenol, 2-Hydroxymethyl-4-amino-phenol, 2-Methoxymethyl-4-amino-phenol, 2-Aminomethyl-4-amino-phenol, 2-(β-Hydroxyethylaminomethyl)-4-amino-phenol und den Additionssalzen dieser Verbindungen mit einer Säure.

4. Zusammensetzung nach einem der vorhergehenden Ansprüche, dadurch gekennzeichnet, daß die m-Aminophenole der Formel (III) ausgewählt sind unter: 5-Amino-2-methoxyphenol, 5-Amino-2-(β-hydroxyethyloxy)-phenol, 5-Amino-2-methyl-phenol, 5-N-(β-Hydroxyethyl)amino-2-methylphenol, 5-N-(β-Hydroxyethyl)amino-4-methoxy-2-methylphenol, 5-Amino-4-methoxy-2-methyl-phenol, 5-Amino-4-chlor-2-methyl-phenol, 5-Amino-2,4-dimethoxyphenol, 5-(γ-Hydroxypropylamino)-2-methyl-phenol und den Additionssalzen dieser Verbindungen mit einer Säure.

5. Zusammensetzung nach einem der vorhergehenden Ansprüche, dadurch gekennzeichnet, daß die Additionssalze mit einer Säure unter den Hydrochloriden, Hydrobromiden, Sulfaten und Tartraten ausgewählt sind.

6. Zusammensetzung nach einem der vorhergehenden Ansprüche, dadurch gekennzeichnet, daß das 2-Amino-3-hydroxypyridin und/oder sein Additionssalz oder seine Additionssalze mit einer Säure 0,0001 bis 10 Gew.-% des Gesamtgewichts der Färbemittelzusammensetzung ausmachen.

7. Zusammensetzung nach Anspruch 6, dadurch gekennzeichnet, daß das 2-Amino-3-hydroxy-pyridin und/oder sein Additionssalz oder seine Additionssalze mit einer Säure 0,005 bis 7 Gew.-% des Gesamtgewichts der Färbemittelzusammensetzung ausmachen.

8. Zusammensetzung nach einem der vorhergehenden Ansprüche, dadurch gekennzeichnet, daß das p-Phenylendiamin oder die p-Phenylendiamine der Formel (I) und/oder das p-Aminophenol oder die p-Aminophenole der Formel (II) 0,0005 bis 10 Gew.-% des Gesamtgewichts der Färbemittelzusammensetzung ausmachen.

9. Zusammensetzung nach Anspruch 8, dadurch gekennzeichnet, daß das p-Phenylendiamin oder die p-Phenylendiamine der Formel (I) und/oder das p-Aminophenol oder die p-Aminophenole der Formel (II) 0,01 bis 7 Gew.-% des Gesamtgewichts der Färbemittelzusammensetzung ausmachen.

10. Zusammenseuzung nach einem der vorhergehenden Ansprüche, dadurch gekennzeichnet, daß das m-Aminophenol oder die m-Aminophenole der Formel (III) 0,0001 bis 5 Gew.-% des Gesamtgewichts der Färbemittelzusammensetzung ausmachen.

11. Zusammensetzung nach Anspruch 10, dadurch gekennzeichnet, daß das m-Aminophenol oder die m-Aminophenole der Formel (III) 0,001 bis 3 Gew.-% des Gesamtgewichts der Färbemittelzusammensetzung ausmachen.

12. Zusammensetzung nach einem der vorhergehenden Ansprüche, dadurch gekennzeichnet, daß das zum Färben geeignete Medium aus Wasser oder einem Gemisch aus Wasser und mindestens einem organischen Lösungsmittel besteht, das unter den niederen $C_{1-4}$-Alkanolen, Glycerin, den Glykolen und Glykolethern, den aromatischen Alkoholen, analogen Produkten und deren Gemischen ausgewählt ist.

13. Zusammensetzung nach einem der vorhergehenden Ansprüche, dadurch gekennzeichnet, daß sie einen pH-Wert im Bereich von 5 bis 12 aufweist.

14. Zusammensetzung nach einem der vorhergehenden Ansprüche, dadurch gekennzeichnet, daß sie ferner weitere Kuppler, die von 2-Amino-3-hydroxy-pyridin, den m-Aminophenolen der Formel (III) und ihren Additionssalzen mit einer Säure verschieden sind, und/oder weitere Oxidationsbasen, die von den p-Phenylendiaminen der Formel (I), den p-Aminophenolen der Formel (II) und ihren Additionssalzen mit einer Säure verschieden sind, und/oder Direktfarbstoffe enthält.

15. Verfahren zum Färben von Keratinfasern und insbesondere menschlichen Keratinfasern, wie dem Haar, dadurch gekennzeichnet, daß auf die Fasern eine Färbemittelzusammensetzung nach einem der Ansprüche 1 bis 14 aufgetragen und die Farbe bei einem sauren, neutralen oder alkalischen pH-Wert mit einem Oxidationsmittel entwikkelt wird, das bei der Anwendung zu dieser Färbemittelzusammensetzung gegeben wird oder das in einer oxidierenden Zusammensetzung vorliegt, die gleichzeitig oder getrennt davon anschließend aufgetragen wird.

16. Verfahren nach Anspruch 15, dadurch gekennzeichnet, daß das in der oxidierenden Zusammensetzung vorliegende Oxidationsmittel unter Wasserstoffperoxid, Harnstoffperoxid, Alkalimetallbromaten und Salzen von Persäuren, wie Perboraten und Persulfaten, ausgewählt ist.

17. Vorrichtung mit mehreren Abteilungen oder "Kit" zum Färben mit mehreren Abteilungen, wobei eine erste Abteilung eine Färbemittelzusammensetzung nach einem der Ansprüche 1 bis 14 und eine zweite Abteilung eine oxidierende

Zusammensetzung mit einem Oxidationsmittel enthält.